# EUROPEAN PATENT APPLICATION

(11) **EP 2 647 620 A1**
(43) Date of publication of application: **09.10.2013**
(21) Application number: 11845569.0
(22) Date of filing: 30.11.2011
(51) Int. Cl.: C07C 229/64, C07C 227/16, C07D 413/04, C07C 227/18

(54) **STABLE SYNTHETIC INTERMEDIATE AND METHOD FOR MANUFACTURING SAME**

(30) Priority: 30.11.2010 JP 2010266687
(71) Applicant: Kyorin Pharmaceutical Co., Ltd., Tokyo 101-8311 (JP)
(72) Inventor: TSUBUKI, Takeshi, Shimotsuga-gun Tochigi 329-0114 (JP); TANASE, Takahiro, Shimotsuga-gun Tochigi 329-0114 (JP); SUZUKI, Masashi, Shimotsuga-gun Tochigi 329-0114 (JP)
(74) Representative: Vossius & Partner
(86) International application number: PCT/JP2011/006697
(87) International publication number: WO 2012/073506

(57) **Abstract**

[Probelm]

To provide a stable industrially-manufactured intermediate, enabling an industrially stable supply of 2-pyridyl-benz[d][1,3]oxazine to be provided.

[Solution]

The compound represented by formula (6) (wherein M represents sodium or potassium).

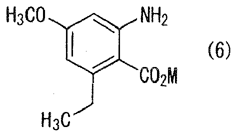

## Description

### Field

The present invention relates to a synthetic intermediate of a benz[d][1,3]oxazine derivative and a method for manufacturing the same.

### Background

Human neutrophil elastase is a kind of serine hydrolase released from granules of neutrophil, which appear during infection or inflammatory disease. Neutrophil elastase is an enzyme hydrolyzing elastin, collagen, proteoglycan, fibronectin, and other proteins which constitute the interstitium of intravital connective tissues such as lung, cartilage, vascular wall, and skin. In addition, it has been clarified that neutrophil elastase acts on other proteins or cells.

Serine hydrolase including neutrophil elastase maintains homeostasis in the living body. The activity of serine hydrolase is controlled by endogenous protein inhibitor such as α1-protease inhibitor, α2-macrogloblin, and secretory leukocyte protease inhibitor. However, when a balance between neutrophil elastase and endogenous inhibitor is lost by excessive release of neutrophil elastase in the inflammation site or by decline in an inhibitor level, the control of neutrophil elastase activity cannot be maintained, and tissues are injured.

Examples of diseases involved in serine hydrolase include pulmonary emphysema, acute respiratory distress syndrome, adult respiratory distress syndrome (ARDS), idiopathic interstitial pneumonia (IIP), cystic pulmonary fibrosis, chronic interstitial pneumonia, chronic bronchitis, chronic sinopulmonary infection, diffuse panbronchiolitis, bronchiectasis, asthma, pancreatitis, nephritis, hepatic failure, rheumatoid arthritis, joint scleroma, osteoarthritis, psoriasis, periodontitis, atherosclerosis, rejection against organ transplant, premature amniorrhexis, bullous dermatosis, shock, sepsis, systemic lupus erythematosus (SLE), Crohn's disease, disseminated intracapillary coagulation (DIC), tissue injury after ischemia-reperfusion, formation of cornea cicatricial tissue, myelitis and the like. As a therapeutic agent for these diseases, the development of serine hydrolase inhibitor is expected.
2-pyridyl-benz[d][1,3]oxazine derivatives have been reported as an excellent serine hydrolase inhibitor (Patent Literature 1). A method for manufacturing 2-pyridyl-benz[d][1,3]oxazine disclosed in Patent Literature 1 is as follows.

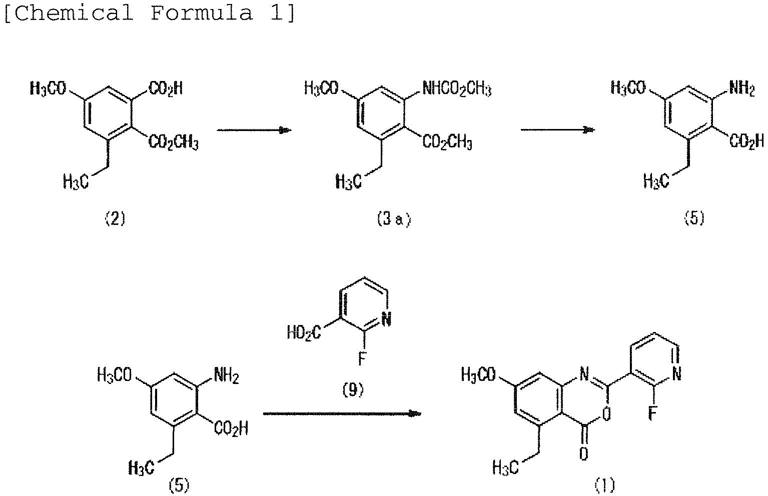

### Citation List

### Patent Literature

Patent Literature 1: WO 2008/036379

### Summary

### Technical Problem

As described above, a compound represented by the formula (1) (hereinafter also referred to as compound (1)) is synthesized by using an anthranilic acid derivative represented by the formula (5) (hereinafter also referred to as compound (5)) as a raw material.

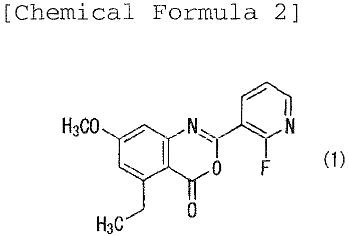

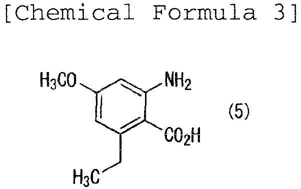

However, it was revealed that the compound (5) is unstable to long-term storage and heating. Thus, it is considered to be unsuitable for an intermediate of the compound (1) in industrial manufacturing.

An object of the present invention is to develop a method for manufacturing the compound (1) using a stable intermediate in industrial manufacturing and to stably supply the compound (1).

### Solution to Problem

The present inventors have investigated the instability of the compound (5), and as a result, found that the compound (5) is degraded by decarboxylation during long-term storage. The inventors have studied in order to find a compound which serves as a raw material for the compound (1) and is difficult to cause decarboxylation, and as a result, found that a particular alkali metal salt of the compound (5) satisfies the required conditions. Thus, the present invention has been completed.

The summary of the present invention is as follows:
1) A compound represented by the formula (6). 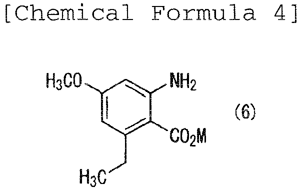 (In the formula (6), M represents sodium or potassium.)
2) A method for manufacturing a compound represented by the formula (6), comprising the following steps (A) to (C).
   (A) reacting a compound represented by the formula (2) with diphenyl phosphoryl azide in a solvent in the presence of a base, followed by a reaction with ROH (In the formula, R represents an alkyl group having one to four carbon atoms) to produce a compound represented by the formula (3);
   (B) hydrolyzing the compound represented by the formula (3) obtained by the step (A) in a solvent using a base, adding 2-propanol and water to the reaction solution, and further adding an acid to precipitate a compound represented by the formula (5); and
   (C) adding an aqueous solution of MOH (In the formula, M represents sodium or potassium) to a 2-propanol solution of the compound represented by the formula (5) obtained by the step (B) to precipitate the compound represented by the formula (6). 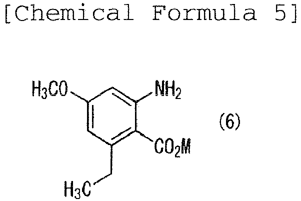 (In the formula (6), M represents sodium or potassium.) 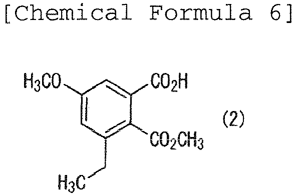 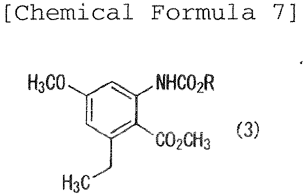 (In the formula (3), R represents an alkyl group having one to four carbon atoms.) 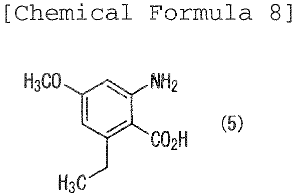
3) A method for manufacturing a compound represented by the formula (1) comprising condensing and cyclizing a compound represented by the formula (6) and a compound represented by the formula (9) using 1-(3-dimethylaminopropyl)-3-ethylcarbodiimide hydrochloride as a condensation agent, to thereby obtain the compound represented by the formula (1). 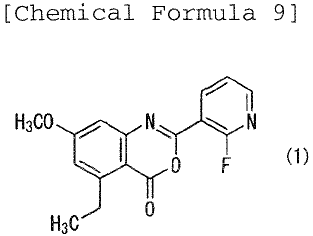 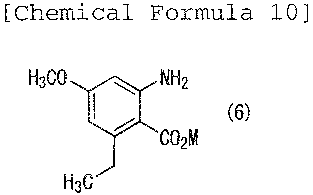 (In the formula (6), M represents sodium or potassium.) 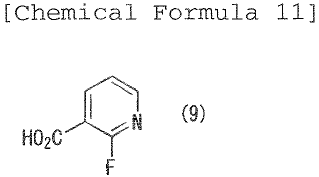
Further, the compound represented by the formula (6) obtained by the method for manufacturing the compound represented by the formula (6), described in 2), can be used. Advantageous Effects of Invention

The present invention provides a method for manufacturing 2-pyridyl-benz[d][1,3]oxazine using a stable intermediate in industrial manufacturing. Thus, the compound can be sustainably supplied.

### Description of Embodiments

In the present invention, 2-pyridyl-benz[d][1,3]oxazine is industrially supplied with sustainability by using the compound represented by the formula (6) (hereinafter also referred to as compound (6)).

### Regarding compound (6)

In the present invention, the compound (6) is a sodium salt or a potassium salt. From the viewpoint of efficiency of filtration operation in manufacturing of the compound (6), a sodium salt is preferable because of a rapid filtration rate. Further, since the compound (6) has high crystallinity, purification by recrystallization is easy. Since a crystal of the compound (6) is difficult to deliquesce, the compound (6) is easily handled in air.
As shown in Reference Example 3 described below, the compound (5) is decarboxylated to release a carbon dioxide during storage at room temperature and degraded into the compound (8). After 10 weeks of storage at room temperature, 50% or more of the compound (5) is degraded (see Test Example 1). The compound (5) has a low stability to heat, and therefore it cannot be subjected to a reaction under a high temperature condition. In addition, it is necessary to avoid heating when the compound (5) is dissolved in a solvent for recrystallization.

On the other hand, the compound (6) is a very stable compound. For example, in Test Example (2), the amount of the compound (8) is only 0.7 % after 19 months of storage of the sodium salt of the compound (6) at room temperature. In Examples 3 and 4, the compound (6) is prepared from the compound (2) as a starting material, and a high yield of 79% is achieved although it is produced through three steps. In a step of converting the compound (5) into a sodium salt, stirring under heating at 50 to 60°C for 1 hour and drying of the compound (6) at 50°C are performed. Thus, it is easy to assume that the compound (6) is also stable to heat.

### Method for manufacturing compound (6)

For example, the compound (6) can be prepared by dissolving the compound (5) in a solvent and treating with a base.
Examples of the solvent which can be used in the present step include ethanol and 2-propanol, and 2-Propanol is preferable. Examples of the base which can be used in the step include: an inorganic base such as an alkali metal hydroxide (for example, sodium hydroxide and potassium hydroxide), an alkali metal carbonate (for example, sodium carbonate and potassium carbonate), and an alkali metal hydrogen carbonate (for example, sodium hydrogen carbonate and potassium hydrogen carbonate), and an aqueous solution thereof; and an organic base such as an alkali metal alkoxide (for example, sodium methoxide, sodium ethoxide, sodium t-butoxide, potassium methoxide, potassium ethoxide, and potassium t-butoxide), and an alcohol solution thereof. The base used in the step is preferably sodium hydroxide, an aqueous solution thereof, potassium hydroxide, or an aqueous solution thereof, more preferably an aqueous solution of sodium hydroxide. Although it is not particularly limited, to smoothly promote the reaction, it is preferable that the reaction is done preferably at 0 to 30°C, and more preferably at 10 to 20°C.

The compound (5) to be used is preferably prepared by the following method to increase the yield and chemical purity of the compound (6).

### (Step A)

The compound represented by the formula (2) is reacted with diphenyl phosphoryl azide in a solvent in the presence of a base, followed by performing a reaction with ROH (In the formula, R represents an alkyl group having one to four carbon atoms) to thereby prepare a compound represented by the formula (3).

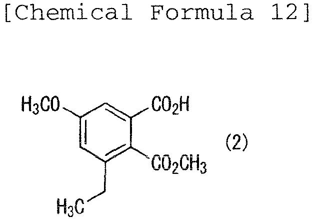

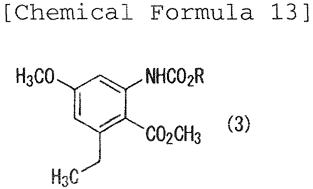

(In the formula (3), R represents an alkyl group having one to four carbon atoms.)

Examples of the solvent which can be used in the step include a nonpolar solvent having a boiling point equal to or higher than the reaction temperature, such as benzene, toluene, and xylene. Examples of the base which can be used include a trialkylamine having a boiling point equal to or higher than the reaction temperature, such as triethylamine. Examples of the alcohol to be reacted include methanol, ethanol, and n-propanol. From the viewpoint of yield of the compound (5), ethanol is preferable.
To s smoothly promote the reaction, the reaction of the compound (2) with diphenyl phosphoryl azide for preparing an acid azide is performed preferably at 10 to 35°C, and more preferably at 20 to 25°C, although it is not particularly limited. Also, to smoothly promote the reaction, the following thermal rearrangement reaction is performed in the presence of the alcohol to be reacted at preferably at 60 to 90°C, and more preferably at 70 to 80°C, although it is not particularly limited.

### (Step B)

The compound represented by the formula (3) is hydrolyzed using a base in a solvent. Subsequently, to the reaction solution, 2-propanol and water are added, and an acid is further added to precipitate a compound represented by the formula (5).

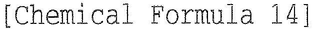

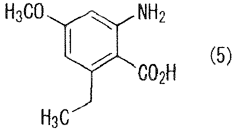

The solvent which can be used in the step is preferably a mixed solvent of water and an organic solvent. Examples of the organic solvent include tetrahydrofuran, methanol, and dimethylsulfoxide. Dimethylsulfoxide is preferable.
Examples of the base which can be used include lithium hydroxide, sodium hydroxide, and potassium hydroxide. Among them, sodium hydroxide is preferable.
Examples of the acid which can be used include hydrochloric acid, sulfuric acid, and phosphoric acid. Among them, hydrochloric acid is preferable.
The hydrolysis is performed preferably at 50 to 100°C, and more preferably at 70 to 80°C, to smoothly promote the reaction, although it is not particularly limited.

The compound (1) can be prepared by condensation and cyclization of the compound (6) and a compound represented by the formula (9).

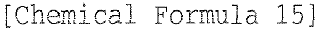

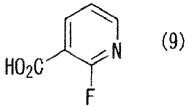

For example, 1-(3-dimethylaminopropyl)-3-ethylcarbodiimide hydrochloride can be used as a condensation agent in the reaction in which the compound (6) and the compound represented by the formula (9) are condensed and cyclized.
Examples of combinations of reagents used in the condensation include p-toluenesulfonyl chloride and 1-methylimidazole, 1,1'-carbonyl diimidazole and 1-(3-dimethylaminopropyl)-3-ethylcarbodiimide hydrochloride, or 1-(3-dimethylaminopropyl)-3-ethylcarbodiimide hydrochloride and 1-hydroxybenzotriazole. Among them, a combination of 1-(3-dimethylaminopropyl)-3-ethylcarbodiimide hydrochloride and 1-hydroxybenzotriazole is preferable from the viewpoint of yield of the compound (1).
Examples of the used solvent include N,N-dimethylformamide, N-methylpyrrolidone, and N,N-dimethylimidazolidinone. N,N-dimethylformamide is preferable. The reaction is performed preferably at 10 to 50°C, and more preferably at 25 to 35°C, to smoothly promote the reaction, although it is not particularly limited.

Hereinafter, the present invention will be described in detail by Examples and Test Examples, and the present invention is not limited to these Examples and Test Examples.

### Example 1 Synthesis of methyl carbamate (3a)

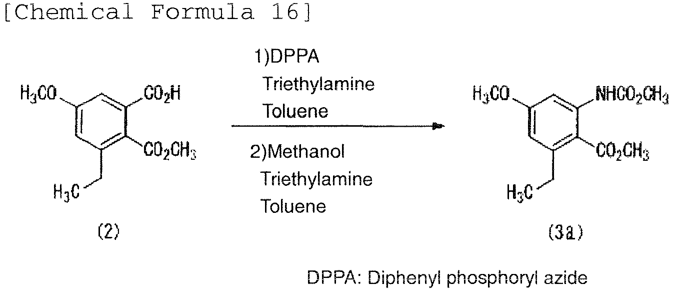

The compound (2) (200 g) was added to toluene (1.0 L), and the mixture was suspended and stirred under ice-cooling. Triethylamine (122 mL) was added dropwise at an inner temperature of 7 to 15°C and The compound (2) was dissolved. Diphenyl phosphoryl azide (236 mL) was added dropwise at an inner temperature of 9 to 13°C and the reaction mixture was stirred at an inner temperature of 20 to 25°C for 2 hours.
Toluene (1.0 L), methanol (170 mL), and triethylamine (122 mL) were placed in another reaction container. The former reaction mixture was added dropwise at an inner temperature of 69 to 75°C over 1 hour under heating while stirring. The container was washed with toluene (400 mL), which was combined with the reaction mixture. The combined mixture was stirred at an inner temperature of 72 to 75°C for 2 hours, and then allowed to stand overnight at room temperature. Iced water (2.0 L) and dilute hydrochloric acid (mixed solution of 50 mL of 36% hydrochloric acid and 150 mL of water) were added under ice-cooling while stirring to adjust the pH to 5. The mixture was allowed to stand, and then an organic layer containing a compound (3a) was separated. The organic layer was concentrated under reduced pressure, and dried under reduced pressure at an outer temperature of 60°C to obtain the compound (3a) (238 g) as a yellow oil.
Instrument data of compound (3a)
¹H-NMR (400 MHz, CDCl₃) δ: 1.18 (3H, t, J = 7.3 Hz), 2.79 (2H, q, J = 7.3 Hz), 3.77 (3H, s), 3.85 (3H, s), 3.90 (3H, s), 6.49 (1H, d, J = 2.4 Hz), 7.79 (1H, d, J = 2.4 Hz), 9.54 (1H, br s). EI-MS m/z: 267 (M⁺).

### Example 2 Synthesis of compound (6) from methyl carbamate (3a)

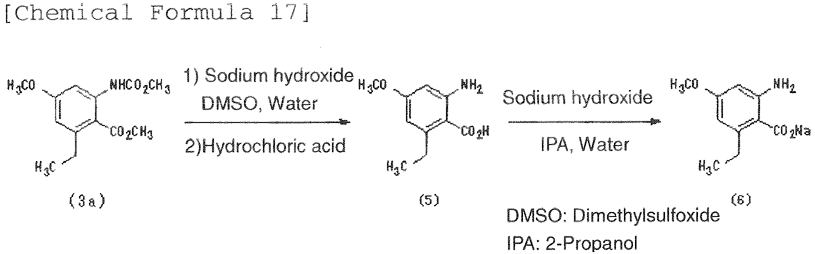

### Step of obtaining compound (5) from compound (3a)

Dimethylsulfoxide (1.30L) was added to the compound (3a) (238 g) and the compound (3a) was dissolved to prepare a solution of the compound (3a). Water (660 mL) and sodium hydroxide (201 g) were added to the solution and the mixture was stirred at an inner temperature of 90 to 98°C for 3 hours and the mixture was allowed to stand overnight at room temperature. To the reaction mixture, 2-propanol (1.3 L) and water (4 L) were added to dissolve the undissolved substance. Then, dilute hydrochloric acid (mixed solution of 469 mL of 36% hydrochloric acid and 1.4 L of water) was added dropwise to the mixture under ice-cooling while stirring at an inner temperature of 5 to 13°C to precipitate the compound (5). The pH of the reaction solution was then adjusted to 4.3 (amount of used dilute hydrochloric acid: 1.62 L). The reaction mixture was stirred at an inner temperature of 5°C for 15 minutes. The precipitated crystal was collected by filtration, and washed with a mixed solution of 2-propanol (60 mL) and water (980 mL), and then deliquored to obtain a wet crystal of the compound (5) (214 g) as a pale yellow powder.

### Step of obtaining compound (6) from compound (5)

The obtained wet crystal of the compound (5) (214 g) was added to 2-propanol (2.65 L) and dissolved under stirring to prepare a solution of the compound (5). 400 g/L sodium hydroxide solution (84 mL) was added dropwise to the solution at an inner temperature of 14 to 17°C to precipitate the compound (6) as a crystal. Then, the reaction mixture was stirred under heating at an inner temperature of 50 to 53°C for 30 minutes. The reaction mixture was stirred under cooling and then stirred at an inner temperature of 8 to 15°C for 30 minutes. Then, the precipitated crystal was collected by filtration. The precipitated crystal was washed with 2-propanol (663 mL), and then deliquored to obtain a wet crystal of the compound (6) (173 g). The obtained wet crystal of the compound (6) was dried with air flow at 50°C overnight to obtain 144 g of the compound (6) as a pale yellow powder (overall yield from the compound (2) : 72%).
Instrument data of compound (6)
Melting point (hot plate method): 236 to 239°C (degradation) Water content (Karl Fischer method): 8.06%
¹H-NMR (400 MHz, DMSO-d₆) δ: 1.06 (3H, t, J = 7.3 Hz), 2.78 (2H, q, J = 7.3 Hz), 3.61 (3H, s), 5.84 (2H, br s), 5.84 (1H, d, J = 2.4 Hz), 5.94 (1H, d, J = 2.4 Hz).

### Example 3 Synthesis of ethyl carbamate (3b)

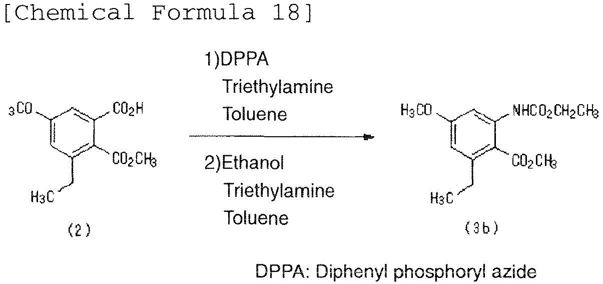

A solution of the compound (2) (5.00 g), toluene (20 mL), and triethylamine (2.23 g) was stirred under cooling. Diphenyl phosphoryl azide (7.52 g) was added dropwise to the solution at an inner temperature of 4°C. The container was then washed with toluene (5 mL), and washing was combined with the reaction mixture. The combined reaction mixture was stirred at an inner temperature of 26 to 27°C for 2 hours.
Toluene (25 mL), ethanol (4.83 g), and triethylamine (2.23 g) were placed in another reaction container. The former reaction mixture was added dropwise at an inner temperature of 70 to 73°C over 1.5 hours under heating while stirring. The container was then washed with toluene (10 mL), which was combined with the reaction mixture. The combined mixture was stirred at an inner temperature of 70 to 72°C for 3 hours, and allowed to stand overnight at room temperature. Water (50 mL) and 3 mol/L hydrochloric acid (5 mL) were added to adjust the pH to 6. An organic layer containing a compound (3b) was separated. The organic layer was concentrated under reduced pressure, and dried under reduced pressure at an outer temperature of 60°C to obtain the compound (3b) (7.43 g) as a colorless oil.
Instrument data of compound (3b)
¹H-NMR (400 MHz, CDCl₃) δ: 1.18 (3H, t, J = 7.6 Hz), 1.32 (3H, t, J = 7.1 Hz), 2.79 (2H, q, J = 7.6 Hz), 3.84 (3H, s), 3.91 (3H, s), 4.21 (2H, q, J = 7.1 Hz), 6.48 (1H, d, J = 2.7 Hz), 7.81 (1H, d, J = 2.4 Hz), 9.50 (1H, br s).

### Example 4 Synthesis of compound (6) from ethyl carbamate (3b)

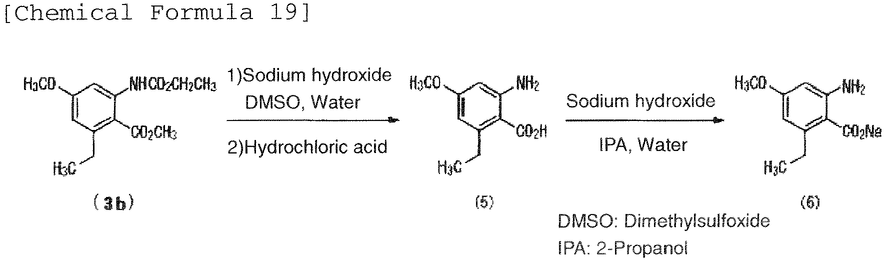

### Step of obtaining compound (5) from compound (3b)

Dimethylsulfoxide (25 mL) was added to the compound (3b) (7.43 g) and the compound (3b) was dissolved to prepare a solution of the compound (3b). A solution of sodium hydroxide (5.04 g) and water (25 mL) was added to the solution of the compound (3b) and the mixture was stirred at an inner temperature of 70 to 80°C for 2 hours. The reaction solution was cooled to an inner temperature of 50°C, and water (50 mL) was added to dissolve a precipitated solid. Then, the resulting reaction mixture was allowed to stand overnight at room temperature. 2-propanol (10 mL) was added to the reaction mixture. Dilute hydrochloric acid (40.5 mL, it is mixed solution which was made from 11.5 mL of 36% hydrochloric acid and 35 mL of water) was added dropwise to the mixture at an inner temperature of 17 to 19°C under stirring to precipitate a compound (5). The pH of the reaction mixture was then adjusted to 4.6. Then, the reaction mixture was cooled and stirred at an inner temperature of 9 to 10°C for 30 minutes. The precipitated crystal was collected by filtration, and washed with a mixed solution of 2-propanol (4 mL) and water (40 mL), and then deliquored to obtain a wet crystal of the compound (5) (4.58 g).

### Step of obtaining compound (6) from compound (5)

2-Propanol (50 mL) was added to the wet crystal of the compound (5) (4.58 g), and the compound (5) was dissolved under stirring to prepare a solution of the compound (5). To the solution, 10 mol/L aqueous sodium hydroxide solution (2.1 mL) was added dropwise at an inner temperature of 21 to 24°C to precipitate a compound (6) as a crystal. Then, the mixture was stirred under heating at an inner temperature of 50 to 60°C for 1 hour. The reaction solution was cooled, and stirred at an inner temperature of 14 to 15°C for 30 minutes. Then, the precipitated crystal was collected by filtration. The precipitated crystal was washed with 2-propanol (15 mL), and deliquored. The resulting crystal was dried with air flow at 50°C to obtain the compound (6) (3.91 g) as a white powder (overall yield from the compound (2): 79%).
Instrument data of compound (6)
Melting point (hot plate method): 237 to 239°C (degradation) Water content (Karl Fischer method): 8.02%
¹H-NMR (400 MHz, DMSO-d₆) δ: 1.07 (3H, t, J = 7.3 Hz), 2.78 (2H, q, J = 7.3 Hz), 3.62 (3H, s), 5.84 (2H, br s), 5.84 (1H, d, J = 2.4 Hz), 5.95 (1H, d, J = 2.4 Hz).

### Example 5 Synthesis of compound (7)

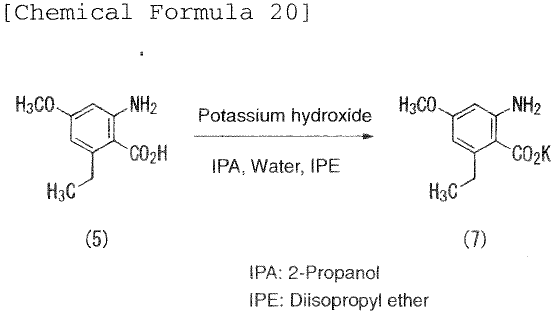

The compound (5) (196 mg) was added to a solution of potassium hydroxide (61.6 mg) in a mixed solution of 2-propanol (2 mL) and water (0.2 mL), and dissolved under heating while stirring at an outer temperature of 55°C to prepare a reaction mixture (pH after the dissolution: 5). Potassium hydroxide (20.9 mg) was further added to the reaction mixture and dissolved at an outer temperature of 55°C (pH after the dissolution: 9). Diisopropyl ether (2 mL) was added to the reaction mixture under water-cooling while stirring to crystallize the compound (7). Then, diisopropyl ether (4 mL) and water (0.1 mL) were added to the reaction mixture and the mixture was stirred under heating at an outer temperature of 55°C. The reaction mixture was stirred under water-cooling. Then, the precipitated crystal was collected by filtration, and washed with 2-propanol (2 mL). The crystal was dried with air flow at 50°C to obtain the compound (7) (193 mg) with a yield of 83% as a light brown flocculent crystal.
Instrument data of compound (7)
¹H-NMR (400 MHz, DMSO-d₆) δ: 1.05 (3H, t, J = 7.3 Hz), 2.75 (2H, q, J = 7.3 Hz), 3.60 (3H, s), 5.72 (2H, br s), 5.82 (1H, d, J = 2.4 Hz), 5.92 (1H, d, J = 2.7 Hz).
FAB-MS(posi) m/z: 131 (base peak), 234 (C₁₀H₁₂KNO₃ + 1).

### Example 6 Synthesis of compound (1) 1

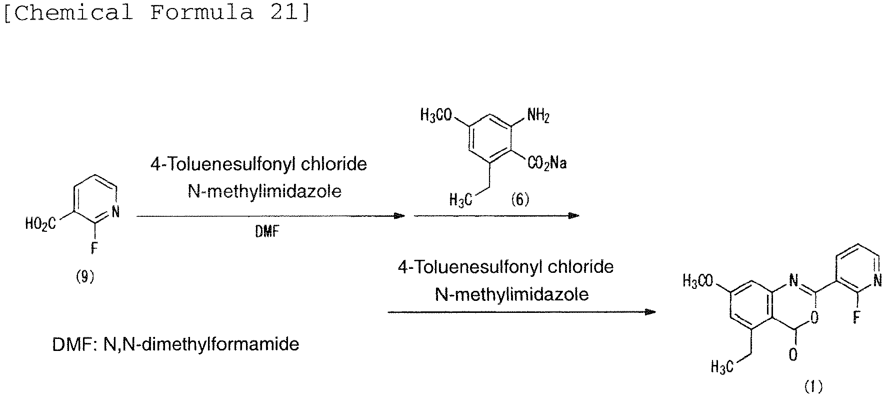

The compound (9) (14.2 g) and 4-toluenesulfonyl chloride (23.2 g) were dissolved in N,N-dimethylformamide (300 mL) to prepare a reaction mixture. N-methylimidazole (24.8 g) was added dropwise to the reaction mixture at an inner temperature of 1 to 8°C under cooling while stirring. The mixture was stirred at an inner temperature of 2 to 14°C for 2.5 hours. A compound (6) (20.0 g: water content: 7.77%) was added to the reaction mixture and the mixture was stirred at an inner temperature of 2 to 12°C for 1.5 hours. Further, 4-toluenesulfonyl chloride (9.62 g) and N-methylimidazole (4.14 g) were added to the reaction mixture and the mixture was stirred at an inner temperature of 8 to 14°C for 3 hours and allowed to stand overnight at room temperature. Water (600 mL) was added dropwise to the reaction mixture at an inner temperature of 21 to 25°C to precipitate a crystal of the compound (1). Then, the mixture was stirred at an inner temperature of 12 to 15°C for 30 minutes. The precipitated crystal was collected by filtration, and washed with water (200 mL), and deliquored. The obtained wet crystal of the compound (1) (23.1 g) was dried with air flow at 50°C to obtain a crude crystal of the compound (1) (17.1 g) as pale yellow powder.
Acetone (800 mL) was added to the crude crystal of the compound (1) (17.1 g) and the compound (1) was dissolved under heating while stirring.
Water (800 mL) was added dropwise to the solution of the compound (1) in acetone at an inner temperature of 46 to 47°C to precipitate a crystal of the compound (1). Then, the mixture was stirred at an inner temperature of 46°C for 10 minutes, and further stirred at an inner temperature of 11 to 15°C for 30 minutes. The precipitated crystal was collected by filtration, and washed with a mixed solution of acetone (100 mL) and water (100 mL), and deliquored. The obtained wet crystal of the compound (1) (23.9 g) was dried with air flow at 50°C to obtain the compound (1) (16.4 g) with a yield of 64.8% as a white powder.
Instrument data of compound (1)
Melting point (hot plate method): 141 to 143°C
¹H-NMR (400 MHz, DMSO-d₆) δ: 1.20 (3H, t, J = 7.3 Hz), 3.11 (2H, q, J = 7.3 Hz), 3.93 (3H, s), 7.03 (2H, s), 7.57 - 7.61 (1H, m), 8.48 - 8.50 (1H, m), 8.55 - 8.60 (1H, m).
HPLC relative purity: 99.5% (RT 42.7 min)
Column: Inertsil ODS-3, 4.6 mm I.D. × 150 mm
Precolumn: Inertsil ODS-3, 4.0 mm I.D. × 10 mm
Measurement wavelength: 260 nm
Column temperature: 35°C
Flow rate: 1.0 mL/min
Mobile phase A: acetonitrile
Mobile phase B: diluted phosphoric acid (1→1, 000) containing 5 mmol/L sodium 1-octanesulfonate

This diluted phosphoric acid (1→1,000) means that 1 mL of phosphoric acid is dissolved in water to make 1,000 mL of solution.

### Mobile phase condition

**[Table 1]**

| TIME AFTER INJECTION (MIN) | MOBILE PHASE A (%) | MOBILE PHASE B (%) |
|---|---|---|
| 0∼18 | 30 | 70 |
| 18∼30 | 30→40 | 70→60 |
| 30∼50 | 40→70 | 60→30 |
| 50∼55 | 70 | 30 |

### Example 7 Synthesis of compound (1) 2

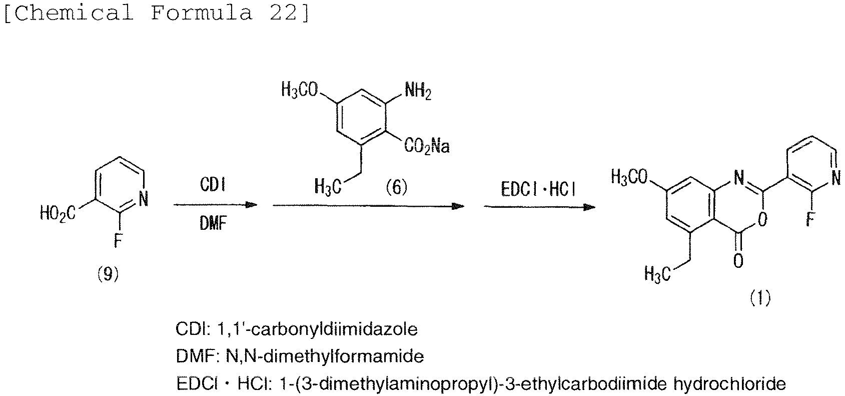

N,N-Dimethylformamide (105 mL) and 1,1'-carbonyldiimidazole (6.68 g) were added to the compound (9) (4.98 g) to prepare a reaction mixture. The reaction mixture was stirred at an inner temperature of 25°C for 1 hour. To the reaction mixture, 1,1'-carbonyldiimidazole (6.68 g) was added again and the mixture was stirred at an inner temperature of 25°C for 40 minutes. To the reaction mixture, the compound (6) (7.00 g : water content: 7.77%) was added and the mixture was stirred at an inner temperature of 26 to 27°C for 2 hours and 20 minutes. Subsequently, 1-(3-dimethylaminopropyl)-3-ethylcarbodiimide hydrochloride (5.64 g) was added to the reaction mixture and the reaction mixture was stirred at an inner temperature of 26°C for 50 minutes. Further, 1-(3-dimethylaminopropyl)-3-ethylcarbodiimide hydrochloride (5.64 g) was added again to the reaction mixture and the reaction mixture was stirred at an inner temperature of 26°C for 1 hour and 15 minutes, and allowed to stand overnight at room temperature. Moreover, 1-(3-dimethylaminopropyl)-3-ethylcarbodiimide hydrochloride (5.64 g) was added again to the reaction mixture under stirring at room temperature, and the reaction mixture was stirred at an inner temperature of 24°C for 9 hours, and allowed to stand overnight at room temperature. Water (210 mL) was added dropwise to the reaction mixture at an inner temperature of 24 to 32°C under stirring at room temperature to precipitate a crystal of the compound (1). Then, the mixture was stirred at an inner temperature of 6 to 15°C for 30 minutes. The precipitated crystal was collected by filtration, and washed with water (70 mL), and deliquored. The resulting wet crystal of the compound (1) (11.5 g) was dried with air flow at 50°C to obtain a crude crystal of the compound (1) (6.02 g) as a light yellow powder.

Acetone (140 mL) was added to the crude crystal of the compound (1) (3.01 g) and the crude crystal was dissolved under heating while stirring. Water (140 mL) was added dropwise to the solution of the compound (1) in acetone at an inner temperature of 45 to 46°C to precipitate a crystal of the compound (1). Then, the mixture was stirred at an inner temperature of 14 to 16°C for 30 minutes. The precipitated crystal was collected by filtration, and washed with a mixed solution of acetone (17.5 mL) and water (17.5 mL), and deliquored. The resulting wet crystal of the compound (1) (2.28 g) was dried with air flow at 50°C to obtain the compound (1) (2.28 g) with a yield of 51.6% as a yellowish white powder.
Instrument data of compound (1)
Melting point (hot plate method): 140 to 141°C
¹H-NMR (400 MHz, DMSO-d₆) δ: 1.20 (3H, t, J = 7.3 Hz), 3.11 (2H, q, J = 7.3 Hz), 3.93 (3H, s), 7.04 (2H, s), 7.57-7.61 (1H, m), 8.48-8.50 (1H, m), 8.55-8.60 (1H, m).
HPLC relative purity: 99.1% (RT 10.8 min)
Column: Inertsil ODS-3, 4.6 mm I.D. × 150 mm
Precolumn: Inertsil ODS-3, 4.0 mm I.D. × 10 mm Measurement wavelength: 260 nm
Column temperature: 30°C
Flow rate: 1.0 mL/min
Mobile phase A: acetonitrile
Mobile phase B: diluted phosphoric acid (1→1,000) containing 5 mmol/L sodium 1-octanesulfonate

### Mobile phase condition

**[Table 2]**

| TIME AFTER INJECTION (MIN) | MOBILE PHASE A (%) | MOBILE PHASE B (%) |
|---|---|---|
| 0∼30 | 55 | 45 |

### Example 8 Synthesis of compound (1) 3

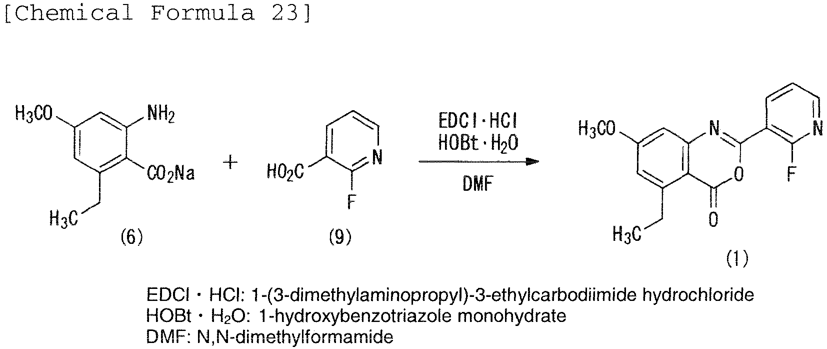

The compound (9) (100 g) and 1-hydroxybenzotriazole monohydrate (109 g) were dissolved in 2.20 L of N,N-dimethylformamide under stirring to prepare a reaction mixture. The compound (6) (167 g : water content: 7.77%) was added to the reaction mixture. Subsequently, 1-(3-dimethylaminopropyl)-3-ethylcarbodiimide hydrochloride (408 g) was added to the reaction mixture at an inner temperature of 21 to 31°C over 40 minutes. The container was washed with N,N-dimethylformamide (300 mL), then the washing was combined. The mixture was stirred at an inner temperature of 22 to 25°C for 3 hours. A solution of sodium hydrogen carbonate (59.5 g) and water (1.20 L) was added to the reaction mixture, water (3.80 L) was then added dropwise, and the mixture was stirred at an inner temperature of 28°C for 30 minutes. The precipitated crystal was collected by filtration, and washed with a mixed solution of acetone (1.00 L) and water (1.00 L), to obtain a wet crystal of crude crystal of the compound (1) (270 g).
The wet crystal of crude crystal of the compound (1) (270 g) was suspended in acetone (7.00 L) while stirring, and the crystal was then dissolved under heating (dissolved at an inner temperature of 36°C). Water (7.00 L) was added dropwise to the solution of the compound (1) in acetone at an inner temperature of 42 to 50°C to precipitate a crystal of the compound (1). Then, the mixture was stirred at an inner temperature of 44 to 45°C for 10 minutes, cooled, and stirred at an inner temperature of 26 to 30°C for 30 minutes. The precipitated crystal was collected by filtration, and washed with a mixed solution of acetone (1.00 L) and water (1.00 L). Then, the obtained wet crystal (236 g) was dried with air flow at 50°C to obtain the compound (1) (168 g) with a yield of 78.9% as a white powder.
Instrument data of compound (1)
Melting point (hot plate method): 142 to 143°C
Elementary analysis Calcd for C₁₆H₁₃FN₂O₃: C, 64.00%; H, 4.36%; N, 9.33%. Found: C, 63.84%; H, 4.63%; N, 9.41%.
EI-MS m/z: 300 [(M + H)⁺. base peak].
¹H-NMR (400 MHz, DMSO-d₆) δ: 1.20 (3H, t, J = 7.3 Hz), 3.11 (2H, q, J = 7.3 Hz), 3.93 (3H ,s), 7.03 (2H, s), 7.57 - 7.61 (1H, m), 8.48 - 8.50(1H, m), 8.55 - 8.60(1H,m).
HPLC relative purity: 99.8% (RT 42.9 min)
Column: Inertsil ODS-3, 4.6 mm I.D. × 150 mm
Precolumn: Inertsil ODS-3, 4.0 mm I.D. × 10 mm
Measurement wavelength: 260 nm
Column temperature: 35°C
Flow rate: 1.0 mL/min
Mobile phase A: acetonitrile
Mobile phase B: diluted phosphoric acid (1→1,000) containing 5 mmol/L sodium 1-octanesulfonate

### Mobile phase condition

**[Table 3]**

| TIME AFTER INJECTION (MIN) | MOBILE PHASE A (%) | MOBILE PHASE B (%) |
|---|---|---|
| 0∼18 | 30 | 70 |
| 18∼30 | 30→40 | 70→60 |
| 30∼50 | 40→70 | 60→30 |
| 50∼55 | 70 | 30 |

### Comparative Example 1 Synthesis of compound (1) 4

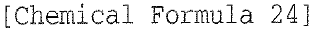

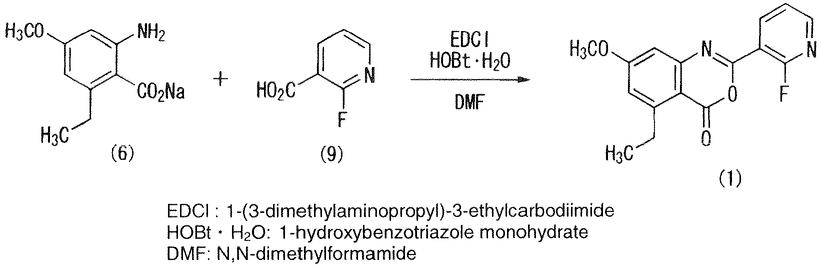

The compound (9) (3.00 g) and 1-hydroxybenzotriazole monohydrate (3.26 g) were dissolved in N,N-dimethylformamide (66.0 mL) under stirring to prepare a reaction mixture. The compound (6) (5.01 g : water content: 7.77%) was added to the reaction mixture. Subsequently, 1-(3-dimethylaminopropyl)-3-ethylcarbodiimide (9.90 g) was added dropwise to the reaction solution at an inner temperature of 12 to 14°C. The container was washed with N,N-dimethylformamide (9.00 mL), then the washing was combined. The reaction mixture was stirred at an inner temperature of 20 to 27°C for 3 hours. Water (150 mL) was added dropwise to the reaction mixture at an inner temperature of 27 to 32°C to precipitate a crystal. The mixture was stirred at an inner temperature of 27 to 30°C for 30 minutes. The precipitated crystal was collected by filtration, and washed with a mixed solution of acetone (30.0 mL) and water (30.0 mL), and then deliquored. The obtained wet crystal (5.31 g) was dried with air flow at 50°C to obtain a reaction mixture (compound (1) and byproduct) (4.62 g) as a light yellow powder.
HPLC relative purity: compound (1) 56.7% (RT 45.3 min) byproduct 35.8% (RT 46.4 min) and 6.5% (RT 52.9 min)
Column: Inertsil ODS-3, 4.6 mm I.D. × 150 mm
Precolumn: Inertsil ODS-3, 4.0 mm I.D. × 10 mm
Measurement wavelength: 260 nm
Column temperature: 35°C
Flow rate: 1.0 mL/min
Mobile phase A: acetonitrile
Mobile phase B: diluted phosphoric acid (1→1,000) containing 5 mmol/L sodium 1-octanesulfonate

### Mobile phase condition

**[Table 4]**

| TIME AFTER INJECTION (MIN) | MOBILE PHASE A (%) | MOBILE PHASE B (%) |
|---|---|---|
| 0∼18 | 30 | 70 |
| 18∼30 | 30→40 | 70→60 |
| 30∼50 | 40→70 | 60→30 |
| 50∼55 | 70 | 30 |

### Reference Example 1 Synthesis of methyl carbamate (3a) 2

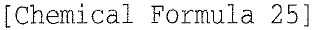

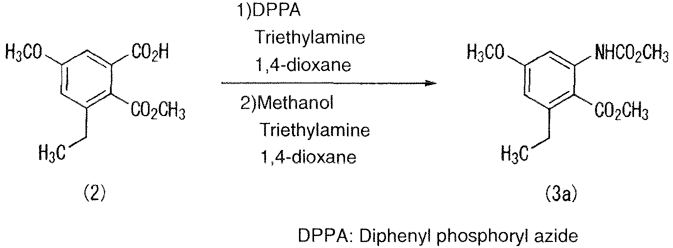

1,4-dioxane (49 mL) and triethylamine (6.0 mL) were added to the compound (2) (9.80 g) and the compound (2) was dissolved, and diphenyl phosphoryl azide (13.4 mL) was then added dropwise at an inner temperature of 23 to 32°C. Then, the mixture was stirred at an inner temperature of 23 to 32°C for 30 minutes. 1,4-dioxane (49 mL), methanol (8.3 mL), and triethylamine (6.0 mL) were placed in another reaction container. The former reaction mixture was added dropwise to the mixture at an inner temperature of 81 to 87°C over 30 minutes under heating while stirring. The container was then washed with 1,4-dioxane (19.6 mL), and washing was combined with the reaction mixture. Then, the mixture was stirred at an inner temperature of 87 to 89°C for 1 hour. The mixture was cooled at room temperature. The reaction mixture was then poured to iced water (300 mL), and was extracted with ethyl acetate (80 mL × 3). Organic layers were combined, washed successively with 0.5 mol/L hydrochloric acid (200 mL), 10% aqueous sodium hydrogen carbonate solution (200 mL), and 10% salt solution (200 mL), and dried with anhydrous sodium sulfate. The undissolved substance was filtered off. The filtrate was concentrated under reduced pressure to obtain the compound (3a) (11.9 g) as a yellow oil. Instrument data of compound (3a)
¹H-NMR (400 MHz, CDCl₃) δ: 1.18 (3H, t, J = 7.3 Hz), 2.79 (2H, q, J = 7.3 Hz), 3.77 (3H, s), 3.84 (3H, s), 3.90 (3H, s), 6.49 (1H, d, J = 2.4 Hz), 7.79 (1H, d, J = 2.4 Hz), 9.54 (1H, brs). Reference Example 2 Synthesis of compound (5)

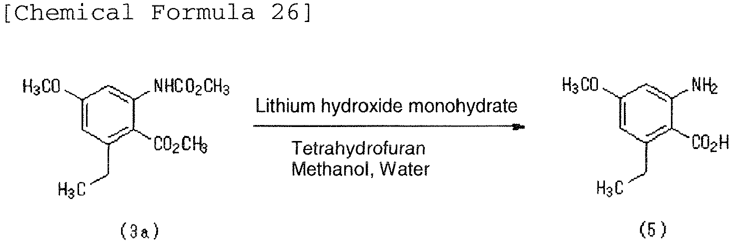

The compound (3a) (11.9 g) was dissolved in a mixed solution of tetrahydrofuran (22 mL) and methanol (22 mL) to obtain a solution of the compound (3a). A solution of lithium hydroxide monohydrate (8.63 g) in water (66 mL) was added to the solution of the compound (3a), and the mixture was stirred at an inner temperature of 69 to 70°C for 1 hour. The reaction mixture was cooled. To the reaction mixture, dilute hydrochloric acid (80 mL, it is mixed solution which made from 18.9 mL of 36% hydrochloric acid and 75.5 mL of water) was added dropwise to adjust the pH to 4.8. The reaction solution was stirred at an inner temperature of 3 to 10°C for 30 minutes. The precipitated crystal was collected by filtration, and washed with water (44 mL), and deliquored. The resulting wet crystal of the compound (5) was dried at 40°C under reduced pressure for 8 hours to obtain the compound (5) (5.28 g) as a white powder (overall yield from the compound (2): 66%).
Instrument data of compound (5)
Melting point (hot plate method): 108 to 109°C (degradation) Elemental Analysis Calcd. for C₁₀H₁₃O₃: C, 61.53; H, 6.71; N, 7.18. Found: C, 61.43; H, 6.64; N, 7.08.
EI-MS m/z : 195 (M⁺).
¹H-NMR (400 MHz, CDCl₃) δ: 1.23 (3H, t, J = 7.3 Hz), 2.93 (2H, q, J = 7.3 Hz), 3.79 (3H, s), 6.01 (1H, d, J = 2.4 Hz), 6.18 (1H, d, J = 2.4 Hz).

### Reference Example 3 Isolation of compound (8) and Confirmation of structure thereof

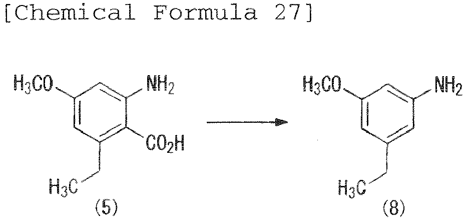

The compound (5) synthesized in Reference Example 2 was sealed in a glass bottle and stored at room temperature. After 10 weeks of storage, the physical properties were changed. A part (500 mg) of the compound (5) was subjected to column purification (silica gel = 20 g, hexane : ethyl acetate = 4 : 1) to obtain the compound (8) (361 mg) which was a degraded substance as a colorless oil.
Instrument data of compound (8)
¹H-NMR (CDCl₃) δ: 1.20 (3H, t, J = 7.8 Hz), 2.52 (2H, q, J = 7.8 Hz), 3.61 (2H, br s), 3.75 (3H, s), 6.08 (1H, t, J = 2.0 Hz), 6.16 (1H, t, J = 1.5 Hz), 6.19 (1H, d, J = 2.0 Hz). EI-MS m/z: 151 (M⁺).

### Test Example 1 Change in physical properties of compounds (5) and (6)

The compound (5) was sealed in a glass bottle and stored at room temperature for 10 weeks. Change in the physical properties is shown in Table 5. The compound (6) was sealed in a glass bottle and stored at room temperature for 19 months. Change in the physical properties is shown in Table 6.

**[Table 5]**

| Compound (5) | IN SYNTHESIS | AFTER 10 WEEKS |
|---|---|---|
| PHYSICAL PROPERTIES | WHITE POWDER | YELLOW SEMI-SOLID |

**[Table 6]**

| Compound (6) | IN SYNTHESIS | AFTER 19 MONTHS |
|---|---|---|
| PHYSICAL PROPERTIES | LIGHT BROWN | LIGHT BROWN |
| | CRYSTALLINE | CRYSTALLINE |
| | POWDER | POWDER |

After 10 weeks of storage, the physical properties of the compound (5) were changed from a white powder to a yellow semi-solid. Even after 19 months of storage, the crystalline powder state of the compound (6) was maintained, and change in the physical properties was not observed.
Therefore, the compound (6) in which the instability of the compound (5) is improved can be stored as a production intermediate. Accordingly, the compound (6) has more advantageous physical properties compared to the compound (5). Test Example 2 Stabilities of compounds (5) and (6)

The compounds (5) and (6) were each sealed in a glass bottle and stored at room temperature. At this time, the chemical purity of each compound was measured. After 6 weeks of storage and after 10 weeks of storage, the compound (5) was measured. After 19 months of storage, the compound (6) was measured. The chemical purity was represented by proportions of each compound and a degraded substance (compound (8)), calculated from area ratios measured by HPLC. HPLC measurement conditions are shown below. The chemical purity of the compound (6) was calculated on the basis of the peak of the compound (5).
HPLC Measurement Conditions
Measurement wavelength: 220 nm
Column: TSKgel Octyl-80Ts, 4.6 mm I.D. × 150 mm
Column temperature: 40°C
Flow rate: 1.0 mL/min
Mobile phase A: acetonitrile
Mobile phase B: diluted phosphoric acid (1→1,000)

### Mobile phase condition

**[Table 7]**

| TIME AFTER INJECTION (MIN) | MOBILE PHASE A (%) | MOBILE PHASE B (%) |
|---|---|---|
| 0∼30 | 20→80 | 80→20 |
| 30∼40 | 80 | 20 |

### HPLC purity of compound (5)

**[Table 8]**

| PEAK NAME | HPLC PURITY | |
|---|---|---|
| | AFTER 6 WEEKS | AFTER 10 WEEKS |
| Compound (5) | 94.1% | 38.4% |
| Compound (8) | 4.1% | 57.8% |

### HPLC purity of compound (6)

**[Table 9]**

| PEAK NAME | HPLC PURITY |
|---|---|
| | AFTER 19 MONTHS |
| Compound (5) | 99.2% |
| Compound (8) | 0.7% |

After 10 weeks of storage of the compound (5), degraded substance (compound (8)) of more than 50% was confirmed. In contrast, even after 19 months of storage of the compound (6), degraded substance of only 0.7% (compound (8)) was confirmed.

### Industrial Applicability

The present invention provides a method for manufacturing 2-pyridyl-benz[d][1,3]oxazine using a stable intermediate in industrial manufacturing, and thus, the sustainable supply of the compound can be enabled.

## Claims

1. A compound represented by the formula (6). 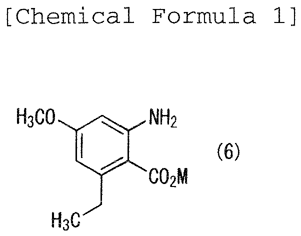 (In the formula (6), M represents sodium or potassium.)

2. A method for manufacturing a compound represented by the formula (6), comprising the following steps (A) to (C).
(A) reacting a compound represented by the formula (2) with diphenyl phosphoryl azide in a solvent in the presence of a base, followed by a reaction with ROH (in the formula, R represents an alkyl group having one to four carbon atoms) to produce a compound represented by the formula (3);
(B) hydrolyzing the compound represented by the formula (3) obtained by the step (A) in a solvent using a base, adding 2-propanol and water to the reaction solution, and further adding an acid to precipitate a compound represented by the formula (5); and
(C) adding an aqueous solution of MOH (in the formula, M represents sodium or potassium) to a 2-propanol solution of the compound represented by the formula (5) obtained by the step (B) to precipitate the compound represented by the formula (6) . 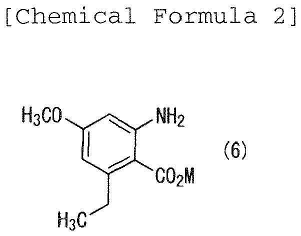 (In the formula (6), M represents sodium or potassium.) 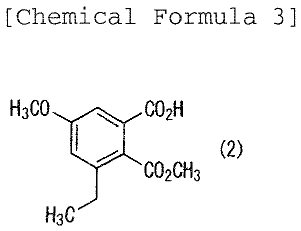 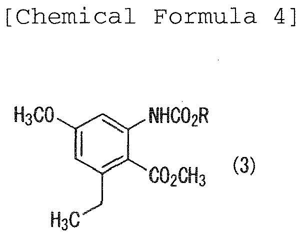 (In the formula (3), R represents an alkyl group having one to four carbon atoms.)

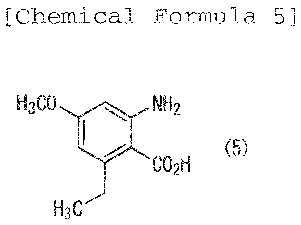

3. A method for manufacturing a compound represented by the formula (1) comprising condensing and cyclizing a compound represented by the formula (6) and a compound represented by the formula (9) using 1-(3-dimethylaminopropyl)-3-ethylcarbodiimide hydrochloride as a condensation agent, to thereby obtain the compound represented by the formula (1). 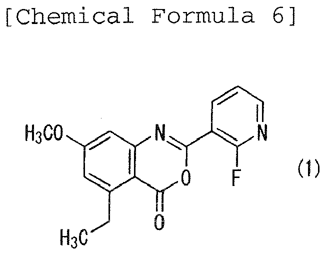 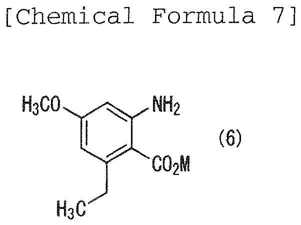 (In the formula (6), M represents sodium or potassium.)
